# Europäisches Patentamt

## European Patent Office

## Office européen des brevets

(19)

(11) Publication number: **0 127 627**
**B1**

(12) # EUROPEAN PATENT SPECIFICATION

(45) Date of publication of the patent specification:
**27.08.86**

(51) Int. Cl.⁴: **D 04 H 1/00**

(21) Application number: **83903236.4**

(22) Date of filing: **14.10.83**

(86) International application number:
**PCT/SE 83/00353**

(87) International publication number:
**WO 84/02363 (21.06.84 Gazette 84/15)**

(54) **A METHOD FOR FORMING A WEB OF MATERIAL AND APPARATUS FOR CARRYING OUT THE METHOD.**

(30) Priority: **10.12.82 SE 8207097**

(73) Proprietor: **JANKEVICS, Verner, Korgpilsgränd 70, S-162 90 VÄLLINGBY (SE)**

(43) Date of publication of application:
**12.12.84 Bulletin 84/50**

(72) Inventor: **JANKEVICS, Verner, Korgpilsgränd 70, S-162 90 VÄLLINGBY (SE)**

(45) Publication of the grant of the patent:
**27.08.86 Bulletin 86/35**

(74) Representative: **Omming, Allan, A. OMMING & CO. AB Sveavägen 28-30, S-111 34 Stockholm (SE)**

(84) Designated Contracting States:
**DE FR GB**

(56) References cited:
**EP - A - 0 072 300**
**SE - B - 169 259**
**US - A - 2 940 133**

# Description

## Technical Field

The present invention relates to a method for forming a web of material by laying particles, for example fibres, distributed in a gaseous medium, on a particle-laying surface, the stream of material comprising said gaseous medium and said particles, being injected in a tangential direction against the inner cylindrical wall of a vessel and being conducted tangentially along the inner cylindrical wall of the vessel, from an inlet to an outlet, from which outlet the particles depart to said particle-laying surface, and to an apparatus for carrying out the method.

## Background Art

The main problem of conducting such a stream of material, in which stream the particles are separate from one another to at least a substantial extend, between the inlet and outlet of said vessel, is one of maintaining the density of the material flow, i.e. of preventing the stream of material from expanding or contracting while, at the same time, maintaining the velocity of the particles substantially constant. It is extremely important that the velocity of the particles is held constant, because if each individual particle is subjected to retardation forces while travelling from the inlet to the region of the outlet of said vessel, the particles will stick together and form aggregates or flocs. When the web formed on the particle-laying surface is a fibre web, these flocs will impair the fluffiness of the web and will result in an uneven product.

A method of laying fibres in web form onto a fibre-laying surface is known, for example, from FR-A-1 135 766. By means of the apparatus described in this Patent Specification the stream of material is led tangentially into a cylindrical drum, in a circular path. Part of the ingoing stream of material is taken out through an outlet in the drum, while the remainder of the stream travels a full revolution and is rejoined with the ingoing stream. The stream of material is greatly contracted during this circulatory movement of the stream, and there is no way of preventing the fibres from adhering to one another and forming flocs. Consequently, a prime object of the present invention is to provide a method of the kind described in the introduction with which the turbulent motion of the stream is maintained without slowing down the particles carried by the gaseous medium to any appreciable extent, while, at the same time, changing the motional mode of the stream of material to a helical movement mode, and an apparatus for carrying out the method.

## Brief disclosure of the invention

The invention is primarily realized by causing the stream of material to flow at a given, selected velocity in said inlet, and, by maintaining a gaseous flow in the axial direction of said vessel, such that said stream of material is guided in a helical path to said region of said outlet, said helical path having a pitch which is selected in dependence upon the velocity in said inlet and the velocity of said axial gas flow.

The particles in the helically moving stream of material will be acted upon by centrifugal forces and by forces created by the central and axial air flow. The gas flow in the said helical path will be so affected by the axial flow of air that its speed will remain constant, or at least substantially constant, and consequently the particles will move at a constant speed throughout the whole of the transport distance, thereby effectively preventing floculation or the like.

## Brief description of the drawings

The invention also relates to apparatus for carrying out the method.

The invention will now be described in more detail with reference to the accompanying drawing, in which

Figure 1 is a simple illustration of the invention

Figure 2 is a top-plan view of apparatus according to the invention, and

Figure 3 illustrates an outlet means for the vessel shown in Figure 2.

## Best mode of carrying out the invention

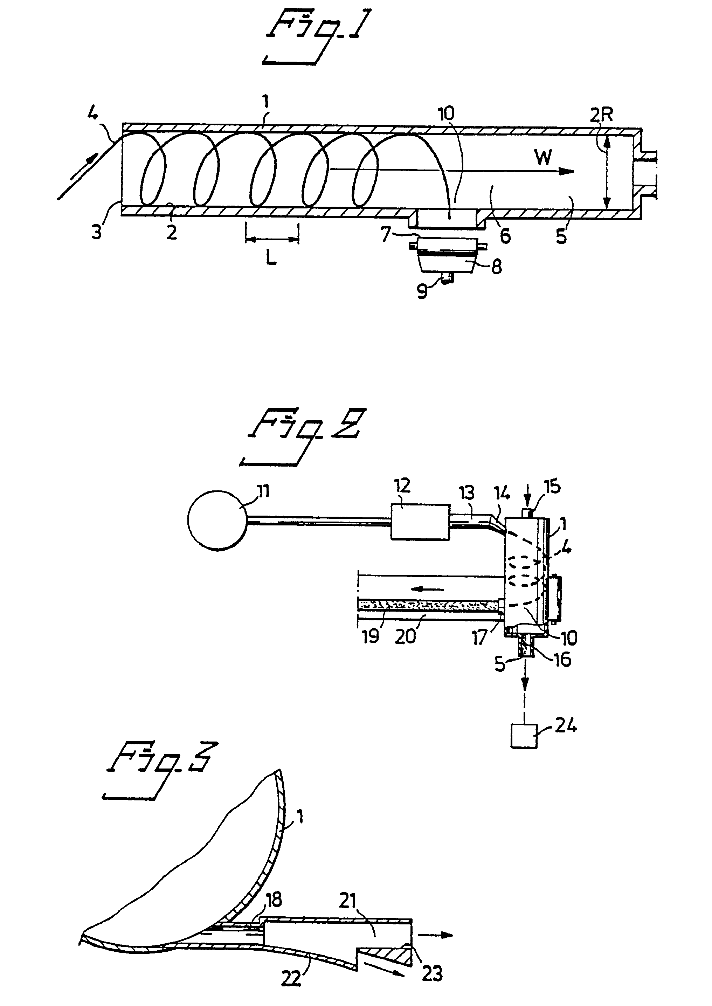

Figure 1 illustrates a vessel 1 having an inner cylindrical wall 2 and an internal cylinder diameter 2R. The vessel 1 has an inlet 3 for a stream of material 4, comprising fibres or other particles uniformly distributed in a gas stream. The stream of material 4 is injected in a tangential direction against the wall 2 at a velocity V, and preferably also at an oblique angle relative to the axis of the cylinder. It is fully possible, however, to feed the stream of material tangentially in a plane extending at right angles to the cylinder axis, where upon the stream of material, unless special measures are taken, will describe circular path, as set forth in the aforementioned FR-A-1 135 766. In accordance with the invention a flow of gas having velocity W is drawn by suction through the vessel 1 in the direction of the cylinder axis, through a suction opening 5. This axial flow of air acts upon the gaseous vehicle of said stream of material, so that said gaseous vehicle describes a rotary movement with laminar flow along the wall 2 and imparts to the gas vortex formed a force which acts axially towards the region of the particle outlet 6. The stream of material 4 is given a desired pitch L within each section of length of the cylinder, in dependence upon the velocity W of the axial air flow. In turn, the velocity W is contingent upon the density of the material stream, the diameter 2R of the vessel and upon the input velocity V. In order to achieve the result intended, i.e. a substantially constant flow velocity in the helical path followed by the stream of material 4 and to maintain a desired, normally variable pitch L along the distance travelled by said material stream, said material stream should have a particle concentration of 50-600 gram fibre/m³ gas and preferably 120-360 grams fibre/m³ gas. With particle concentrations in excess of 600 grams fibre/m³ gas it is necessary to employ extremely high input velocities V, and the effect of the resultant centrifugal forces may then be so great as to cause a certain degree of flocculation in the gas layer located nearest the wall 2. With particle concentration of 50-600 gram fibre/m³ gas it is possible to operate at inlet velocities of the order of 5-200 m/s, preferably 10-50m/s.

The outlet 6 of the vessel 1 is located above a moveable particle-laying surface 7, which is suitably gas-permeable and which is connected to a suction box 8, said suction box being connected to a source of suction (not shown) via a pipe 9. When the stream of material 4 reaches the region 10 adjacent the outlet 6, that part of the axial air flow which has reached this region will be divided into a part air-flow through the suction opening 5 and an air flow out or in through the outlet 6, depending upon the setting of a throttle valve 16, arranged in the outlet 5 and the suction force from the suction box 8 and the pitch L, and the velocity of the material stream will consequently decrease to an extent such that the fibres are moved out through the outlet 6 and laid on the fibre-laying surface 7. Throughout the whole of the transport path between the inlet 3 and said region 10, all fibres in the stream of material 4 have been kept in motion at a substantially unchanged velocity, and have consequently not been able to form flocs. The distance between the point at which retardation is commenced above the outlet 6 and the particle laying surface 7 can be made sufficiently short to totally obviate the risk of flocculation.

The thickness of the stream of material, which may, for example, be up to 80% of the radius of said vessel, can be adjusted by changing the velocity in the helical gas stream, which follows the wall of the vessel, and the axial air flow. The width of the web is determined, for example, by the width of an injection nozzle.

Figure 2 illustrates in a simple manner an apparatus for producing a uniform and homogenous layer of cellulose fibres used as absorbent material in, for example, diapers or sanitary towels. Cellulose fibres are fed pneumatically from a store or a shredder 11 to, for example, a jet ejector 12, from which a stream of gas carrying mutually discrete fibres is fed through a line 13 to an acceleration nozzle 14, by means of which the stream of material 4 is injected into the vessel. In the illustrated embodiment, the vessel 1 has an air inlet 15, and said axial air flow is drawn, by means of a fan 24, through said inlet towards the suction outlet 5, said outlet 5 suitably being provided with said adjustable throttle valve 16 for regulating the axial air flow. The vessel 1 is provided with an outlet pipe 17 which is arranged tangentially relative to the wall of the vessel 1 and which exhibits an outlet gap 18. The outlet gap 18 extends in the axial direction of the vessel 1 and has a length which corresponds to the width of the homogenous fibre web 19, said web being laid on an endless belt 20, which is driven in the direction of the arrow shown. The belt 20 suitably comprises a wire cloth having a suction box arranged therebeneath.

In order to separate out those fibre flocs which are not broken down in the acceleration nozzle, or in some other suitable device, prior to feeding the material to the vessel 1, the aforementioned outlet part may be provided with any known, suitable separating means. A suitable means intended for this purpose is illustrated in Figure 3. Thus, there may be arranged a lower slightly curved surface 22, which the individual fibres follow (the Coanda effect) and a separating tongue 23, over which the fibre flocs, which have a larger mass than an individual fibre and thus greater kinetic energy, are thrown and returned, for example to the shredder 11 via a line 21, while the individual, lightweight fibres are fed to the driven belt 20 and form thereupon a uniform and fully homogenous fibre web, with the fibres lying substantially parallel to one another.

In the aforegoing it has been assumed that the stream of material is fed tangentially into the vessel. Although this is to be preferred, it is also possible to introduce the stream of material axially, into the vessel with the aid of a vortex forming device arranged to impart a rotary movement to the gas stream carrying said fibres. In order to direct stream of material purely axially so that said stream moves tangentially, or at least substantially tangentially, on to the inner wall surface of the cylindrical vessel, it is possible, for example, to use well known techniques with air jets arranged to guide said stream into the desired mode of motion. It is also possible to feed in a plurality of material streams lying helically adjacent one another and having mutually the same pitch. Theoretically, it is possible to place such material streams without mutual spacing therebetween, thereby to provide a continuous layer which moves helically along the inner surface of the vessel.

A further, and particularly important advantage afforded by the invention is that the fibres in the injected gaseous medium will be aligned in the direction of the helical path of said gaseous medium and will therefore have a higher degree of parallelism than fibres laid by means of known apparatus.

In order to eliminate as far as possible friction between the fibres present in the gas stream and the inner surfaces of the vessel 1, said inner surfaces are made as smooth as possible and, for example, are polished. The thickness of the ingoing stream of material and its velocity, and also the pitch of the helix are regulated partly by the velocity of the injected stream and the amount of gas therein, partly by the radius of the vessel, and partly by the velocity of the axial air flow.

## Claims

1. A method of forming a web of material, by laying particles, for example fibres, distributed in a gaseous medium, on a particle-laying surface, the stream of material comprising said gaseous medium and said particles being injected in a tangential direction against the inner cylindrical wall of a vessel and being conducted tangentially along the inner cylindrical wall of the vessel, from an inlet to an outlet, from which outlet the particles depart to said particle-laying surface, characterized by causing the stream of material to flow at a given, selected velocity in said inlet, and, by maintaining a gaseous flow in the axial direction of said vessel, such that said stream of material is guided in a helical path to the region of said outlet, said helical path having a pitch which is selected in dependence upon the velocity in said inlet and the velocity of said axial gas flow.

2. A method according to Claim 1, characterized by accelerating said stream of material in said inlet.

3. A method according to Claim 1 or Claim 2, characterized in that the stream of material has a particle concentration of 120-600 g fibre/m³ gas, preferably 120-360 g fibre/m³ gas.

4. A method according to any one of Claims 1-3, characterized in that the stream of material has a velocity of between 5 and 200 m/s, preferably between 10 and 50 m/s.

5. Apparatus for carrying out the method according to Claim 1, comprising a vessel (1) having an inlet for a stream of material (4), said stream comprising particles distributed in a gas, injection means (12, 13, 14) for feeding said stream in a tangential direction against the inner cylindrical wall of the vessel (1), and an outlet (17) for laying particles onto a particle-laying surface (20), characterized by means (24) for generating an axial flow of gas in the interior of the vessel (1), from the inlet towards the region (10) of the outlet (17).

6. Apparatus according to Claim 5, characterized in that the injection means comprises an acceleration nozzle (14).

7. Apparatus according to Claim 5 or Claim 6, characterized in that the vessel (1) is provided with a suction-outlet opening (5) which is connected to a suction-fan (24); and in that arranged in a suction line extending between the suction-outlet opening (5) and the suction fan (24) is a means (16) for regulating the air flow.

8. Apparatus according to any one of Claims 5-7, characterized in that the outlet (17) is provided with separating means (22, 23) for separating agglomerated particles from individual particles.

## Patentansprüche

1. Verfahren zur Bildung einer Materialbahn durch Auflegen von Teilchen, beispielsweise von Fasern, die in einem gasförmigen Medium verteilt sind, auf eine Teilchen-Auflagefläche, wobei der Materialstrom das gasförmige Medium und die Teilchen enthält, in tangentialer Richtung gegen die innere zylindrische Wand eines Behälters eingeblasen wird und tangential längs der inneren zylindrischen Wand des Behälters von einem Einlass zu einem Auslass geleitet wird, von welchem Auslass aus die Teilchen auf die Teilchen-Auflagefläche austreten, dadurch gekennzeichnet, dass man den Materialstrom veranlasst, mit einer vorgegebenen, ausgewählten Geschwindigkeit im Einlass zu strömen, und dass man eine gasförmige Strömung in axialer Richtung des Behälters so aufrechterhält, dass der Materialstrom in einem wendelförmigen Weg zum Bereich des Auslasses geführt wird, wobei der wendelförmige Weg eine Steigung aufweist, die in Abhängigkeit von der Geschwindigkeit im Einlass und der Geschwindigkeit der axialen Gasströmung gewählt ist.

2. Verfahren nach Anspruch 1, dadurch gekennzeichnet, dass man den Materialstrom im Einlass beschleunigt.

3. Verfahren nach Anspruch 1 oder Anspruch 2, dadurch gekennzeichnet, dass der Materialstrom eine Teilchenkonzentration von 120 bis 600 g Fasern/m³ Gas und bevorzugt 120 bis 360 g Fasern/m³ Gas aufweist.

4. Verfahren nach einem der Ansprüche 1 bis 3, dadurch gekennzeichnet, dass der Materialstrom eine Geschwindigkeit zwischen 5 und 200 m/s und bevorzugt zwischen 10 und 50 m/s aufweist.

5. Vorrichtung zum Durchführen des Verfahrens nach Anspruch 1, mit einem Behälter (1), der einen Einlass für einen Materialstrom (4) aufweist, der in einem Gas verteilte Teilchen aufweist, einer Einblaseinrichtung (12, 13, 14) zum Einleiten des Stromes in einer tangentialen Richtung gegen die innere zylindrische Wand des Behälters (1), und einem Auslass (17) zum Auflegen der Teilchen auf eine Teilchen-Auflagefläche (20), gekennzeichnet durch eine Einrichtung (24) zum Erzeugen einer axialen Gasströmung im Inneren des Behälters (1) vom Einlass zum Bereich (10) des Auslasses (17) hin.

6. Vorrichtung nach Anspruch 5, dadurch gekennzeichnet, dass die Einblaseinrichtung eine Beschleunigungsdüse (14) aufweist.

7. Vorrichtung nach Anspruch 5 oder Anspruch 6, dadurch gekennzeichnet, dass der Behälter (1) mit einer Saug-Auslassöffnung (5) versehen ist, die mit einem Sauggebläse (24) verbunden ist, und dass in einer Saugleitung, die sich zwischen der Saug-Auslassöffnung (5) und dem Sauglüfter (24) erstreckt, eine Einrichtung (16) zum Regulieren der Luftströmung angeordnet ist.

8. Vorrichtung nach irgendeinem der Ansprüche 5 bis 7, dadurch gekennzeichnet, dass der Auslass (17) mit einer Trenneinrichtung (22, 23) versehen ist, um agglomerierte Teilchen von Einzelteilchen abzutrennen.

## Revendications

1. Procédé de formation d'une nappe de matière, par dépôt de particules, par exemple de fibres, réparties dans un milieu gazeux, sur une surface de dépôt de particules, le courant de matière comprenant ce milieu gazeux et ces particules étant injectées dans une direction tangentielle contre la paroi cylindrique interne d'un récipient et étant conduites tangentiellement le long de la paroi cylindrique interne du récipient, de l'orifice d'entrée à un orifice de sortie, de cet orifice de sortie les particules partent vers cette surface de dépôt des particules, caractérisé en ce qu'on amène le courant de matière à s'écouler à une vitesse donnée, choisie, dans cet orifice d'entrée, et en ce que, en maintenant un courant gazeux dans la direction axiale de ce récipient tel que ce courant de matière est guidé sur un trajet hélicoïdal vers la région de cet orifice de sortie, ce trajet hélicoïdal ayant un pas qui est choisi en fonction de la vitesse dans cet orifice d'entrée et de la vitesse de ce courant de gaz axial.

2. Procédé selon la revendication 1, caractérisé en ce qu'on accélère ce courant de matière dans cet orifice d'entrée.

3. Procédé selon les revendications 1 ou 2, caractérisé en ce que le courant de matière a une concentration de particules de 120-600 grammes de fibres/m³ de gaz, de préférence de 120-360 grammes de fibres/m³ de gaz.

4. Procédé selon l'une quelconque des revendications 1 à 3, caractérisé en ce que le courant de matière a une vitesse comprise entre 5 et 200 m/s, de préférence entre 10 et 50 m/s.

5. Appareil pour exécuter le procédé selon la revendication 1, comprenant un récipient (1) ayant un orifice d'entrée pour un courant de matière (4), ce courant comprenant des particules réparties dans un gaz, des moyens d'injection (12, 13, 14) pour envoyer ce courant dans une direction tangentielle contre la paroi cylindrique interne du récipient (1), et un orifice de sortie (17) pour déposer des particules sur une surface de dépôt des particules (20), caractérisé par des moyens (24) pour produire un courant axial de gaz à l'intérieur du récipient 1, depuis l'orifice d'entrée en direction de la région (10) de l'orifice de sortie (17).

6. Appareil selon la revendication 5, caractérisé len ce que le moyen d'injection comprend une buse d'accélération (14).

7. Appareil selon les revendications 5 ou 6, caractérisé en ce que le récipient (1) est muni d'une ouverture de sortie à aspiration (5) qui est reliée à un ventilateur d'aspiration (24); et en ce qu'un moyen (16) pour réguler le courant d'air est disposé dans une canalisation d'aspiration s'étendant entre l'ouverture de sortie avec aspiration (5) et le ventilateur d'aspiration (24).

8. Appareil selon l'une quelconque des revendications 5 à 7, caractérisé en ce que l'orifice de sortie (17) est muni de moyens de séparation (22, 23), pour séparer les particules agglomérées des particules individuelles.

*Fig.1*

*Fig.2*

*Fig.3*